# EUROPEAN PATENT SPECIFICATION

(11) **EP 1 973 912 B1**
(45) Date of publication and mention of the grant of the patent: **15.07.2009**
(21) Application number: 07703697.8
(22) Date of filing: 08.01.2007
(51) Int. Cl.: C07D 471/06

(54) **TETRABENZODIAZADIKETOPERYLENE COLOURANTS IN IR-REFLECTIVE COATINGS AND PLASTICS**
TETRABENZODIAZADIKETOPERYLEN-FÄRBEMITTEL IN IR-REFLEKTIVEN BESCHICHTUNGEN UND KUNSTSTOFFEN
MATIÈRES COLORANTES DE TYPE TÉTRABENZODIAZADIKETOPÉRYLÈNE DANS DES REVÊTEMENTS ET DES PLASTIQUES RÉFLÉCHISSANTS LES RAYONNEMENTS INFRAROUGES

(30) Priority: 18.01.2006 US 759732 P
(43) Date of publication of application: 01.10.2008
(73) Proprietor: Ciba Holding Inc., 4057 Basel (CH)
(72) Inventor: SARVER, Joseph E., Erial, NJ 08081 (US); COLE, Damien Thurber, Drexel Hill, PA 19026 (US); CAMPBELL, Colin Dennis, Claymont, DE 19703 (US)
(86) International application number: PCT/EP2007/050146
(87) International publication number: WO 2007/082811

(56) References cited:
- WO-A-20/05030878
- JP-A- 63 193 960
- US-A- 5 028 643

## Description

The invention belongs to a method for producing infra-red reflective substrates, including molded polymeric articles, films, fibers and coatings and other organic and inorganic materials, by incorporating into the substrate or onto the surface of the substrate an effective amount of a tetrabenzodiazadiketoperylene pigment or dye. The pigments and dyes of the invention have the added property of being transparent to much of the infra-red (IR) radiation not reflected. Thus, very little IR radiation is absorbed by these darkly colored pigments or dyes, further limiting heat build up and allowing for the production of laser welded articles.

Materials which possess infra-red (IR) reflectance characteristics have proven to be valuable in many current applications. Such materials reduce IR-induced heat buildup and find use in automotive and marine coatings, containers, colored plastics such as vinyl siding etc. Thermally robust compositions also find use in inorganic and organic glazing and aerospace, architectural and other glass and ceramic decorative applications where reduced heat buildup is desired.

Other applications which make use of IR reflective materials include protective camouflage for military applications.

US-6,171,383 and US-6,221,147 disclose IR reflective bismuth manganese oxide green pigments with improved heat buildup properties.

US-5,028,643 discloses tetrabenzodiazadiketoperylene pigments and a method for their preparation.

US-6,989,056 discloses IR reflective black pigment compositions containing a halogenated copper phthalocyanine and a perylenetetracarboxylic acid diimide.

In spite of the advancements made in the art, there remains a need for new, stable, IR reflective compositions.

It has durprisingly been found that incorporating tetrabenzodiazadiketoperylene (TBDKP) colourants (pigments or dyes) into plastics or coatings render the plastics or coatings IR reflective. The colourants of the present invention allow for the preparation of dark colored (including black and brown), IR reflective substrates.

As used herein, the term "IR reflectance" means the reflectance properties of a material at wavelengths above about 700 nm. That is, electromagnetic radiation at wavelengths above about 700 nm is reflected. The reflectance characteristics of the articles produced via this invention are highly advantageous in applications where heat buildup due to the absorption of IR radiation is to be minimized and where detection by IR sensors is to be minimized.

The same pigments and dyes, which are darkly colored, for example shades of black and brown, are also found to be transparent to much of the infra-red (IR) radiation not reflected. Incorporating the pigments or dyes into, for example, a layer which is in contact with a substrate containing an IR absorbing material, such as a carbon black pigmented polymer, allows one to pass IR radiation, as from a laser, through the layer containing the pigments of the invention to the underlying substrate generating enough heat at the point of irradiation to "laser weld", or melt the two materials together.

A method is provided for preparing an infra red (IR) reflective organic or inorganic substrate which method comprises the incorporation into the substrate, or the application onto the surface of the substrate, of a composition containing a tetrabenzodiazadiketoperylene pigment or dye (TBDKP) of formula 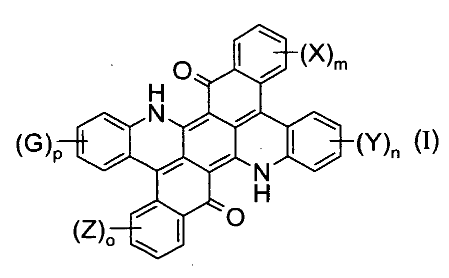 in an amount effective to impart to the organic or inorganic substrate an infra red reflectance of greater than about 50 percent at wavelengths between 800 and 1200 nm, in which formula (I)
X, Y, Z and G independently of each other are linear or branched C₁-C₁₂alkyl, C₃-C₆cycloalkyl, C₇-C₁₂aralkyl, C₆-C₁₀aryl, C₃-C₉ saturated or unsaturated heterocycle, halogen, OR, CF₃, COOR, CONR'R", NO₂, NR'R", SO₃H or SO₂NR'R";
R' and R", independently of each other are hydrogen, linear or branched C₁-C₈alkyl, C₃-C₆cycloalkyl, C₆-C₁₀aryl or C₇-C₁₂aralkyl, and when attached to nitrogen, R' and R" can also, together with the nitrogen atom to which they are attached, form a 5-, 6- or 7-membered ring which is uninterrupted or interrupted by O, NH or N(C₁-C₄alkyl);
and m, n, o and p are independently 0, 1, 2, 3 or 4, and when m, n, o or p is 2, 3 or 4, each X, Y, Z or G substituent is independently from all others a group as defined above.

The inorganic or organic substrate may be, for example, a naturally occurring polymer or a synthetic polymer, for example a thermoplastic, elastomeric, inherently crosslinked or crosslinked polymer. For example, a method which method comprises incorporating into a thermoplastic, elastomeric, crosslinked or inherently crosslinked polymer an amount of a tetrabenzodiazadiketoperylene pigment or dye (TBDKP) of formula (I) effective to impart to the thermoplastic, elastomeric, crosslinked or inherently crosslinked polymer an infra red reflectance of greater than about 50 percent at wavelengths between 800 and 1200 nm.

In particular, X, Y, Z and G independently of each other are linear or branched C₁-C₁₂alkyl, C₇-C₁₂aralkyl, C₆-C₁₀aryl, F, Cl, Br, I, OR', COOR', CONR'R", NO₂, NR'R", SO₃H or SO₂NR'R" and m, n, o and p are independently 0, 1, 2, 3 or 4.

Preferably, X, Y, Z and G independently of each other are linear or branched C₁-C₁₂alkyl, C₇-C₁₂aralkyl, C₆-C₁₀aryl, F, Cl, Br, OR', COOR', CONR'R", NO₂, NR'R", SO₃H or SO₂NR'R" and m, n, o and p are independently 0, 1 or 2.

Particularly preferred, X, Y, Z and G independently of each other are linear or branched C₁-C₁₂alkyl, F, Cl, OR, NO₂, NR'R" or SO₃H and m, n, o and p are independently 0, 1 or 2.

For example, m, n, o and p are each 0 and the tetrabenzodiazadiketoperylene pigment of formula (I) is

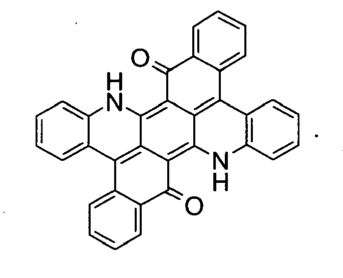

Alkyl or branched alkyl is straight or branched chain of the specified number of carbon atoms and is for example methyl, ethyl, n-propyl, n-butyl, sec-butyl, tert-butyl, n-hexyl, n-octyl, 2-ethylhexyl, n-nonyl, n-decyl, n-undecyl, n-dodecyl.

The composition containing the tetrabenzodiazadiketoperylene pigment or dye may be composed entirely of the pigment or dye, or other materials as disclosed herein may also be present.

The tetrabenzodiazadiketoperylene pigments and dyes of the present invention are prepared according to the methods of US-5,028,643 or PCT/EP2006/068373.

As used herein, the term "IR reflectance" means the reflectance properties of a material at wavelengths above about 700 nm. That is, electromagnetic radiation at wavelengths above about 700 nm is reflected. Substrates containing the present pigments or dyes, or articles coated with compositions containing the present pigments or dyes, typically have an IR reflectance of greater than about 50 percent at wavelengths between 800 and 1200 nm, in particular at about 1000 nm, and have measurable IR reflectance at higher wavelengths, for example, between about 1200 and 2000 nm.

These reflectance characteristics are highly advantageous in applications where heat buildup due to the absorption of IR radiation is to be minimized and where detection by IR sensors is to be minimized.

Typically, the TBDKP pigment or dye is incorporated into a composition comprising a thermoplastic, thermoset, elastomeric, inherently crosslinked or crosslinked polymer. The polymer may be, for example, in the form of a film, sheet, injection-moulded article, extruded workpiece, fiber, laminate, felt or woven fabric. The polymer may also be part of a coating composition.

The TBDKP pigment or dye is either incorporated directly into the substrate, or applied to the surface of the substrate.

When applied to the surface of a substrate, the TBDKP pigment or dye may be part of a coating composition. The coating can comprise any coating system, or even a preformed film, which both adheres to the substrate and is compatible with the TBDKP pigment or dye, for example, auto coatings, marine coatings, paints, inks, laminates, receiving layers for printing applications, or other protective or decorative coatings including coatings or films used in glazing applications. The TBDKP pigment or dye may also be part of a fabric treatment.

Examples of thermoplastic, thermoset, elastomeric, inherently crosslinked or crosslinked polymers into which the pigments or dyes of the present invention may be incorporated into or coated onto are listed below.
1. Polymers of mono- and di-olefins, for example polypropylene, polyisobutylene, polybutene-1, poly-4-methylpentene-1, polyisoprene or polybutadiene and also polymerisates of cyclo-olefins, for example of cyclopentene or norbornene; and also polyethylene (which may optionally be crosslinked), for example high density polyethylene (HDPE), high density polyethylene of high molecular weight (HDPE-HMW), high density polyethylene of ultra-high molecular weight (HDPE-UHMW), medium density polyethylene (MDPE), low density polyethylene (LDPE), and linear low density polyethylene (LLDPE), (VLDPE) and (ULDPE).
   Polyolefins, that is to say polymers of mono-olefins, as mentioned by way of example in the preceding paragraph, especially polyethylene and polypropylene, can be prepared by various processes, especially by the following methods:
   a) by free radical polymerisation (usually at high pressure and high temperature);
   b) by means of a catalyst, the catalyst usually containing one or more metals of group IVb, Vb, VIb or VIII. Those metals generally have one or more ligands, such as oxides, halides, alcoholates, esters, ethers, amines, alkyls, alkenyls and/or aryls, which may be either π- or α-coordinated. Such metal complexes may be free or fixed to carriers, for example to activated magnesium chloride, titanium(III) chloride, aluminium oxide or silicon oxide. Such catalysts may be soluble or insoluble in the polymerisation medium. The catalysts can be active as such in the polymerisation or further activators may be used, for example metal alkyls, metal hydrides, metal alkyl halides, metal alkyl oxides or metal alkyl oxanes, the metals being elements of group(s) Ia, IIa and/or IIIa. The activators may have been modified, for example, with further ester, ether, amine or silyl ether groups.
2. Mixtures of the polymers mentioned under 1.), for example mixtures of polypropylene with polyisobutylene, polypropylene with polyethylene (for example PP/HDPE, PP / LDPE) and mixtures of different types of polyethylene (for example LDPE/HDPE).
3. Copolymers of mono- and di-olefins with one another or with other vinyl monomers, for example ethylene/propylene copolymers, linear low density polyethylene (LLDPE) and mixtures thereof with low density polyethylene (LDPE), propylene/butene-1 copolymers, propylene/isobutylene copolymers, ethylene/butene-1 copolymers, ethylene/hexene copolymers, ethylene/methylpentene copolymers, ethylene/heptene copolymers, ethylene/octene copolymers, propylene/butadiene copolymers, isobutylene/isoprene copolymers, ethylene/alkyl acrylate copolymers, ethylene/alkyl methacrylate copolymers, ethylene/vinyl acetate copolymers and copolymers thereof with carbon monoxide, or ethylene/acrylic acid copolymers and salts thereof (ionomers), and also terpolymers of ethylene with propylene and a diene, such as hexadiene, dicyclopentadiene or ethylidenenorbornene; and also mixtures of such copolymers with one another or with polymers mentioned under 1.), for example polypropylene-ethylene/propylene copolymers, LDPE-ethylene/vinyl acetate copolymers, LDPE-ethylene/acrylic acid copolymers, LLDPE-ethylene/vinyl acetate copolymers, LLDPE-ethylene/acrylic acid copolymers and alternately or randomly structured polyalkylene-carbon monoxide co-polymers and mixtures thereof with other polymers, for example polyamides.
4. Hydrocarbon resins (for example C₅-C₉) including hydrogenated modifications thereof (for example tackifier resins) and mixtures of polyalkylenes and starch.
5. Polystyrene, poly(p-methylstyrene), poly(α-methylstyrene).
6. Copolymers of styrene or α-methylstyrene with dienes or acrylic derivatives, for example styrene/butadiene, styrene/acrylonitrile, styrene/alkyl methacrylate, styrene/butadiene/alkyl acrylate and methacrylate, styrene/maleic anhydride, styrene/acrylonitrile/methyl acrylate; high-impact-strength mixtures consisting of styrene copolymers and another polymer, for example a polyacrylate, a diene polymer or an ethylene/propylene/diene terpolymer; and also block copolymers of styrene, for example styrene/butadiene/styrene, styrene/isoprene/styrene, styrene/ethylene-butylene/styrene or styrene/ethylene-propylene/styrene.
7. Graft copolymers of styrene or α-methylstyrene, for example styrene on polybutadiene, styrene on polybutadiene/styrene or polybutadiene/acrylonitrile copolymers, styrene and acrylonitrile (or methacrylonitrile) on polybutadiene; styrene, acrylonitrile and methyl methacrylate on polybutadiene; styrene and maleic anhydride on polybutadiene; styrene, acrylonitrile and maleic anhydride or maleic acid imide on polybutadiene; styrene and maleic acid imide on polybutadiene, styrene and alkyl acrylates or alkyl methacrylates on polybutadiene, styrene and acrylonitrile on ethylene/propylene/diene terpolymers, styrene and acrylonitrile on polyalkyl acrylates or polyalkyl methacrylates, styrene and acrylonitrile on acrylate/butadiene copolymers, and mixtures thereof with the copolymers mentioned under 6.), such as those known, for example, as so-called ABS, MBS, ASA or AES polymers.
8. Halogen-containing polymers, for example polychloroprene, chlorinated rubber, chlorinated and brominated copolymer of isobutylene/isoprene (halobutyl rubber), chlorinated or chlorosulfonated polyethylene, copolymers of ethylene and chlorinated ethylene, epichlorohydrin homo- and co-polymers, especially polymers of halogen-containing vinyl compounds, for example polyvinyl chloride, polyvinylidene chloride, polyvinyl fluoride, polyvinylidene fluoride; and copolymers thereof, such as vinyl chloride/vinylidene chloride, vinyl chloride/vinyl acetate or vinylidene chloride/vinyl acetate.
9. Polymers derived from α,β-unsaturated acids and derivatives thereof, such as polyacrylates and polymethacrylates, or polymethyl methacrylates, polyacrylamides and polyacrylonitriles impact-resistant-modified with butyl acrylate.
10. Copolymers of the monomers mentioned under 9.) with one another or with other unsaturated monomers, for example acrylonitrile/butadiene copolymers, acrylonitrile/alkyl acrylate copolymers, acrylonitrile/alkoxyalkyl acrylate copolymers, acrylonitrile/vinyl halide copolymers or acrylonitrile/alkyl methacrylate/butadiene terpolymers.
11. Polymers derived from unsaturated alcohols and amines or their acyl derivatives or acetals, such as polyvinyl alcohol, polyvinyl acetate, stearate, benzoate or maleate, polyvinylbutyral, polyallyl phthalate, polyallylmelamine; and the co-polymers thereof with olefins mentioned in 1.).
12. Homo- and co-polymers of cyclic ethers, such as polyalkylene glycols, polyethylene oxide, polypropylene oxide or copolymers thereof with bisglycidyl ethers.
13. Polyacetals, such as polyoxymethylene, and also those polyoxymethylenes which contain comonomers, for example ethylene oxide; polyacetals modified with thermoplastic polyurethanes, acrylates or MBS.
14. Polyphenylene oxides and sulfides and mixtures thereof with styrene polymers or polyamides.
15. Polyurethanes derived from polyethers, polyesters and polybutadienes having terminal hydroxyl groups on the one hand and aliphatic or aromatic polyisocyanates on the other hand, and their initial products.
16. Polyamides and copolyamides derived from diamines and dicarboxylic acids and/or from aminocarboxylic acids or the corresponding lactams, such as polyamide 4, polyamide 6, polyamide 6/6, 6/10, 6/9, 6/12, 4/6, 12/12, polyamide 11, polyamide 12, aromatic polyamides derived from m-xylene, diamine and adipic acid; polyamides prepared from hexamethylenediamine and iso- and/or tere-phthalic acid and optionally an elastomer as modifier, for example poly-2,4,4-trimethylhexamethylene terephthalamide or poly-m-phenylene isophthalamide. Block copolymers of the above-mentioned polyamides with polyolefins, olefin copolymers, ionomers or chemically bonded or grafted elastomers; or with polyethers, for example with polyethylene glycol, polypropylene glycol or polytetramethylene glycol. Also polyamides or copolyamides modified with EPDM or ABS; and polyamides condensed during processing ("RIM polyamide systems").
17. Polyureas, polyimides, polyamide imides, polyether imides, polyester imides, polyhydantoins and polybenzimidazoles.
18. Polyesters derived from dicarboxylic acids and dialcohols and/or from hydroxycarboxylic acids or the corresponding lactones, such as polyethylene terephthalate, polybutylene terephthalate, poly-1,4-dimethylolcyclohexane terephthalate, polyhydroxybenzoates, and also block polyether esters derived from polyethers with hydroxyl terminal groups; and also polyesters modified with polycarbonates or MBS.
19. Polycarbonates and polyester carbonates.
20. Polysulfones, polyether sulfones and polyether ketones.
21. Crosslinked polymers derived from aldehydes on the one hand and phenols, urea or melamine on the other hand, such as phenol-formaldehyde, ureaformaldehyde and melamine-formaldehyde resins.
22. Drying and non-drying alkyd resins.
23. Unsaturated polyester resins derived from copolyesters of saturated and unsaturated dicarboxylic acids with polyhydric alcohols, and also vinyl compounds as crosslinking agents, and also the halogen-containing, difficultly combustible modifications thereof.
24. Crosslinkable acrylic resins derived from substituted acrylic esters, e.g. from epoxy acrylates, urethane acrylates or polyester acrylates.
25. Alkyd resins, polyester resins and acrylate resins that are crosslinked with melamine resins, urea resins, isocyanates, isocyanurates, polyisocyanates or epoxy resins.
26. Crosslinked epoxy resins derived from aliphatic, cycloaliphatic, heterocyclic or aromatic glycidyl compounds, e.g. products of bisphenol-A diglycidyl ethers, bisphenol-F diglycidyl ethers, that are crosslinked using customary hardeners, e.g. anhydrides or amines with or without accelerators.
27. Natural polymers, such as cellulose, natural rubber, gelatin, or polymer-homologously chemically modified derivatives thereof, such as cellulose acetates, propionates and butyrates, and the cellulose ethers, such as methyl cellulose; and also colophonium resins and derivatives.
28. Mixtures (polyblends) of the afore-mentioned polymers, for example PP/EPDM, polyamide / EPDM or ABS, PVC / EVA, PVC /ABS, PVC / MBS, PC / ABS, PBTP / ABS, PC / ASA, PC / PBT, PVC / CPE, PVC/acrylates, POM /thermoplastic PUR, PC /thermoplastic PUR, POM / acrylate, POM / MBS, PPO / HIPS, PPO / PA 6.6 and copolymers, PA/HDPE, PA/PP, PA / PPO, PBT / PC/ABS or PBT / PET / PC.

The thermoplastic, elastomeric, crosslinked or inherently crosslinked polymer is, for example, a polyolefin, polyamide, polyurethane, polyacrylate, polyacrylamide, polyvinyl alcohol, polycarbonate, polystyrene, polyester, polyacetal, a natural or synthetic rubber or a halogenated vinyl polymer such as PVC. The polymer may be a co-polymer, a polymer blend or part of a composite.

The TBDKP pigments and dyes of the instant invention may be incorporated into polymer resins according a variety of known methods. For example, the compounds may be added as an individual component during blending, for example, dry blending of the resin prior to prior to processing, or the compound may be added as a blend, master batch, flush, or other concentrate in another substance prior to processing. The compounds may also be added during processing steps. Standard process steps for polymer resins are well known in the literature and include extrusion, coextrusion, compression molding, Brabender melt processing, film formation, injection molding, blow molding, other molding and sheet forming processes, fiber formation etc.

The compounds of formula (I) are also incorporated via dry blending, surface impregnation, suspension, dispersion and other methods known in coatings technology.

When the pigments or dyes of the instant invention are used in a film, the film is applied to the surface by, for example, the use of an adhesive, or co-extruded onto the surface. A preformed film may also be applied with heat which includes calendaring, melt applications and shrink wrapping.

When a substrate is coated with an IR reflecting coating comprising a TBDKP pigment or dye, the coating typically comprises a polymeric binder which can in principle be any binder customary in industry, for example those described in Ullmann's Encyclopedia of Industrial Chemistry, 5th Edition, Vol. A18, pp. 368-426, VCH, Weinheim 1991. In general, it is a film-forming binder based on a thermoplastic or thermosetting resin, for example, a thermosetting resin. Examples thereof are alkyd, acrylic, acrylamide, polyester, styrenic, phenolic, melamine, epoxy and polyurethane resins.

For example, non-limiting examples of common coating binders useful in the present invention include silicon containing polymers, fluorinated polymers, unsaturated polyesters, unsaturated polyamides, polyimides, crosslinkable acrylic resins derived from substituted acrylic esters, e.g. from epoxy acrylates, urethane acrylates, polyester acrylates, polymers of vinyl acetate, vinyl alcohol and vinyl amine. The coating binder polymers may be co-polymers, polymer blends or composites.

Coatings are frequently crosslinked with, for example, melamine resins, urea resins, isocyanates, isocyanurates, polyisocyanates, epoxy resins, anhydrides, poly acids and amines, with or without accelerators.

The binder can be a cold-curable or hot-curable binder. In many instances it is desirable to use tetrabenzodiazadiketoperylene pigment rather than dye. In these instances, the binder can be a cold-curable or hot-curable binder provided that the temperature is not high enough to cause dissolution of the tetrabenzodiazadiketoperylene pigment; the addition of a curing catalyst may be advantageous. Suitable catalysts which accelerate curing of the binder are described, for example, in Ullmann's Encyclopedia of Industrial Chemistry, 5th Edition, Vol. A18, p.469, VCH Verlagsgesellschaft, Weinheim 1991.

The binder may be a surface coating resin which dries in the air or hardens at room temperature. Exemplary of such binders are nitrocellulose, polyvinyl acetate, polyvinyl chloride, unsaturated polyester resins, polyacrylates, polyurethanes, epoxy resins, phenolic resins, and especially alkyd resins. The binder may also be a mixture of different surface coating resins. Provided the binders are curable binders, they are normally used together with a hardener and/or accelerator.

Examples of coating compositions containing specific binders are:
1. coatings based on cold- or hot-crosslinkable alkyd, acrylate, polyester, epoxy or melamine resins or mixtures of such resins, if desired with addition of a curing catalyst;
2. two-component polyurethane coatings based on hydroxyl-containing acrylate, polyester or polyether resins and aliphatic or aromatic isocyanates, isocyanurates or polyisocyanates;
3. one-component polyurethane coatings based on blocked isocyanates, isocyanurates or polyisocyanates which are deblocked during baking, if desired with addition of a melamine resin;
4. one-component polyurethane coatings based on a Trisalkoxycarbonyltriazine crosslinker and a hydroxyl group containing resin such as acrylate, polyester or polyether resins;
5. one-component polyurethane coatings based on aliphatic or aromatic urethaneacrylates or polyurethaneacrylates having free amino groups within the urethane structure and melamine resins or polyether resins, if necessary with curing catalyst;
6. two-component coatings based on (poly)ketimines and aliphatic or aromatic isocyanates, isocyanurates or polyisocyanates;
7. two-component coatings based on (poly)ketimines and an unsaturated acrylate resin or a polyacetoacetate resin or a methacrylamidoglycolate methyl ester;
8. two-component coatings based on carboxyl- or amino-containing polyacrylates and polyepoxides;
9. two-component coatings based on acrylate resins containing anhydride groups and on a polyhydroxy or polyamino component;
10. two-component coatings based on acrylate-containing anhydrides and polyepoxides;
11. two-component coatings based on (poly)oxazolines and acrylate resins containing anhydride groups, or unsaturated acrylate resins, or aliphatic or aromatic isocyanates, isocyanurates or polyisocyanates;
12. two-component coatings based on unsaturated polyacrylates and polymalonates;
13. thermoplastic polyacrylate coatings based on thermoplastic acrylate resins or externally crosslinking acrylate resins in combination with etherified melamine resins; and
14. paint systems based on siloxane-modified or fluorine-modified acrylate resins.

Acrylic, methacrylic and acrylamide polymers and co-polymers dispersible in water are readily used as a binder in the present invention, for example, acrylic, methacrylic and acrylamide dispersion polymers and co-polymers.

For example, coatings or films comprising acrylate polymers are useful in the instant invention.

The coating composition can also comprise further components, examples being solvents, pigments, dyes, plasticizers, stabilizers, thixotropic agents, drying catalysts and/or levelling agents. Examples of possible components are those described in Ullmann's Encyclopedia of Industrial Chemistry, 5th Edition, Vol. A18, pp. 429-471, VCH, Weinheim 1991.

Possible drying catalysts or curing catalysts are, for example, organometallic compounds, amines, amino-containing resins and/or phosphines. Examples of organometallic compounds are metal carboxylates, especially those of the metals Pb, Mn, Co, Zn, Zr or Cu, or metal chelates, especially those of the metals Al, Ti or Zr, or organometallic compounds such as organotin compounds, for example.

Examples of metal carboxylates are the stearates of Pb, Mn or Zn, the octoates of Co, Zn or Cu, the naphthenates of Mn and Co or the corresponding linoleates, resinates or tallates.

Examples of metal chelates are the aluminium, titanium or zirconium chelates of acetylacetone, ethyl acetylacetate, salicylaldehyde, salicylaldoxime, o-hydroxyacetophenone or ethyl trifluoroacetylacetate, and the alkoxides of these metals.

Examples of organotin compounds are dibutyltin oxide, dibutyltin dilaurate or dibutyltin dioctoate.

Examples of amines are, in particular, tertiary amines, for example tributylamine, triethanolamine, N-methyldiethanolamine, N-dimethylethanolamine, N-ethylmorpholine, N-methylmorpholine or diazabicyclooctane (triethylenediamine) and salts thereof. Further examples are quaternary ammonium salts, for example trimethylbenzylammonium chloride.

Amino-containing resins are simultaneously binder and curing catalyst. Examples thereof are amino-containing acrylate copolymers.

The curing catalyst used can also be a phosphine, for example triphenylphosphine.

The coating compositions can also be radiation-curable coating compositions. In this case, the binder essentially comprises monomeric or oligomeric compounds containing ethylenically unsaturated bonds, which after application are cured by actinic radiation, i.e. converted into a crosslinked, high molecular weight form.

Where the system is UV-curing, it generally contains a photoinitiator as well. Corresponding systems are described in the abovementioned publication Ullmann's Encyclopedia of Industrial Chemistry, 5th Edition, Vol. A18, pages 451-453. In radiation-curable coating compositions, the novel stabilizers can also be employed without the addition of sterically hindered amines.

The coating may also be a radiation-curable, solvent-free formulation of photopolymerisable compounds. Illustrative examples are mixtures of acrylates or methacrylates, unsaturated polyester / styrene mixtures or mixtures of other ethylenically unsaturated monomers or oligomers.

The coating compositions can comprise an organic solvent or solvent mixture in which the binder is soluble. The coating composition can otherwise be an aqueous solution or dispersion. The vehicle can also be a mixture of organic solvent and water. The coating composition may be a high-solids paint or can be solvent-free (e.g. a powder coating material). Powder coatings are, for example, those described in Ullmann's Encyclopedia of Industrial Chemistry, 5th Edition, Vol. A18, pages 438-444. The powder coating material may also have the form of a powder-slurry (dispersion of the powder preferably in water).

Multilayer systems are possible where the tetrabenzodiazadiketoperylene pigment or dye may reside in a coating which is then itself coated with another coating, such as a protective coating.

When used in a coating, the compounds of formula (I) are incorporated into the coating via techniques common in the art.

The coating composition according to the invention can be applied to any desired substrate, for example to metal, wood, plastic, composite, glass or ceramic material substrates by the customary methods, for example by brushing, spraying, pouring, draw down, spin coating, dipping or electrophoresis; see also Ullmann's Encyclopedia of Industrial Chemistry, 5th Edition, Vol. A18, pp. 491-500.

The TBDKP pigment or dye is present in the IR-reflective composition in an "effective amount", that is an amount that provides both the desired level of coloration for the substrate or coating and also provides the desired IR-reflectance.

For example, the TBDKP pigment or dye is present in an amount of about 0.01 to about 50% by weight of tetrabenzodiazadiketoperylene pigment or dye, based on the total weight of the composition, especially 0.01-15%, preferably 0.1-10% or especially preferred 0.1-5% by weight, based on the total weight of the composition. In the case of a coating, the composition is the fully dried and cured coating.

It is also envisioned that when applied to the surface of a substrate, the TBDKP pigment or dye may be present in even higher amounts, even approaching 100%, such as in a thin layer, or a layer which is part of a laminate structure.

A composition comprising the present TBDKP pigments or dyes may also optionally have incorporated therein or applied thereto other additives such as antioxidants, UV absorbers, hindered amine or other light stabilizers, phosphites or phosphonites, benzofuran-2-ones, thiosynergists, polyamide stabilizers, metal stearates, nucleating agents, fillers, reinforcing agents, lubricants, emulsifiers, dyes, pigments, dispersants, optical brighteners, flame retardants, antistatic agents, blowing agents and the like.

The present invention also provides an IR reflective composition or article comprising an organic or inorganic substrate, typically a polymeric substrate, and an amount of a tetrabenzodiazadiketoperylene pigment or dye of formula (I) effective to impart to the organic or inorganic substrate an infra red reflectance of greater than about 50 percent at wavelengths between 800 and 1200 nm. The IR reflective composition or article may be a coating, film, sheet or molded or otherwise shaped article.

For example, an infra red reflective composition comprising a thermoplastic, elastomeric, crosslinked or inherently crosslinked polymer and an amount of a tetrabenzodiazadiketoperylene pigment or dye (TBDKP) of formula (I) effective to impart to the composition an infra red reflectance of greater than about 50 percent at wavelengths between 800 and 1200 nm.

Also provided is an IR reflective article comprising a substrate which is coated with an IR reflective coating or film comprising a TBDKP pigment or dye of formula (I).

A further embodiment of the invention provides a method for laser welding a layered article wherein a TBDKP pigment or dye of formula (I) is incorporated into a polymeric composition which is in contact with a surface of a meltable substrate, preferably a polymeric substrate, containing an IR absorbing material, such as a carbon black pigmented polymer, then IR radiation, as from a laser, is passed through the layer containing the pigments of the invention to the underlying IR absorbing material generating enough heat at the point of irradiation to "laser weld", that is melt together the two materials.

The lasers used are commonly available lasers which emit at wavelengths between about 700 and about 2000 nm, for example, between about 800 and about 1500 nm.

The Examples that follow illustrate the invention (unless otherwise specified, "%" is always % by weight):
Example 1: A mixture of 1 g of tetrabenzodiazadiketoperylene and 999 g of polypropylene (Profax^{®} 6301, Himont, Wilmington, Del. / US), is extruded using a 35 mm twin-screw extruder at 200°C then injection injection molded into 2 mm thick chips at temperatures of at least 250°C. The IR reflectance of the resulting black chips is measured and found to be between 60 and 80% from 800 to 1200 nm, between 40% and 60% from 1300 to 1400 nm and over 30% at about 1600 nm.
Example 2: The procedure of example 1 is repeated using 1 g of chlorinated tetrabenzodiazadiketoperylene to produce brown polypropylene chips with similar IR reflectance, as in example 1.
Example 3: A mixture of 2.3 g of a toner of tetrabenzodiazadiketoperylene, 1.2 g of DISPERBYK^{®} 161, 16.9 g of an acrylic mill base and 39.3 g of a letdown is milled with 100 g of 2 mm glass beads using a SKANDEX^{®} mill. The resulting paint is separated from the beads.

A drawdown of the paint using a 100 µm wet film wired bar and a KCC automatic film applicator is prepared and dried over a white / black leneta card. The IR reflectance of the chips is measured and found to be between greater than 80% from 800 to 1300 nm, between 60% and 80% from 1300 to 1500 nm, greater than 60% at about 1800 nm, and greater than 50 % at about 2000 nm.

## Claims

1. A method for preparing an infra red (IR) reflective organic or inorganic substrate, comprising the incorporation into the substrate, or the application onto the surface of the substrate, of a composition containing a tetrabenzodiazadiketoperylene (TBDKP) colourant of formula 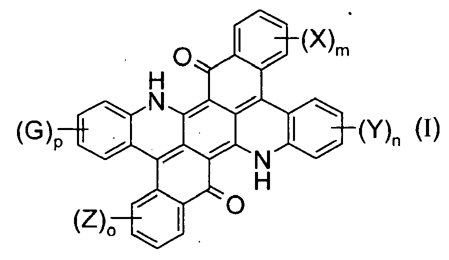
in an amount effective to impart to the organic or inorganic substrate an infra red reflectance of greater than 50 percent at wavelengths between 800 and 1200 nm, in which formula (I)
X, Y, Z and G independently of each other are linear or branched C₁-C₁₂alkyl, C₃-C₆cycloalkyl, C₇-C₁₂aralkyl, C₆-C₁₀aryl, C₃-C₉ saturated or unsaturated heterocycle, halogen, OR, CF₃, COOR, CONR'R", NO₂ NR'R", SO₃H or SO₂NR'R";
R' and R", independently of each other are hydrogen, linear or branched C₁-C₈alkyl, C₃-C₆cycloalkyl, C₆-C₁₀aryl or C₇-C₁₂aralkyl, and when attached to nitrogen, R' and R" can also, together with the nitrogen atom to which they are attached, form a 5-, 6- or 7-membered ring which is uninterrupted or interrupted by O, NH or N(C₁-C₄alkyl);
and m, n, o and p are independently 0, 1, 2, 3 or 4, and when m, n, o or p is 2, 3 or 4, each X, Y, Z or G substituent is independently from all others a group as defined above.

2. A method according to claim 1, wherein the colourant of formula (I) is incorporated into a thermoplastic, elastomeric, crosslinked or inherently crosslinked polymer.

3. A method according to claim 1, wherein X, Y, Z and G independently of each other are linear or branched C₁-C₁₂alkyl, C₇-C₁₂aralkyl, C₆-C₁₀aryl, F, Cl, Br, I, OR', COOR', CONR'R", NO₂, NR'R", SO₃H or SO₂NR'R" and m, n, o and p are independently 0, 1, 2, 3 or 4, preferably wherein X, Y, Z and G independently of each other are linear or branched C₁-C₁₂alkyl, C₇-C₁₂aralkyl, C₆-C₁₀aryl, F, Cl, Br, OR', COOR', CONR'R", NO₂, NR'R", SO₃H or SO₂NR'R" and m, n, o and p are independently 0, 1 or 2, most preferred wherein the colourant is of formula

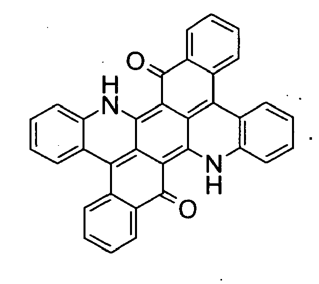

4. The method according to claim 1, wherein the tetrabenzodiazadiketoperylene is incorporated into a coating composition which is applied to the surface of the substrate.

5. The method according to claim 2, wherein the tetrabenzodiazadiketoperylene colourant of formula (I) is a pigment and the polymer is preferably a polyolefin, polyamide, polyurethane, polyacrylate, polyacrylamide, polyvinyl alcohol, polycarbonate, polystyrene, polyester, polyacetal, a natural or synthetic rubber or a halogenated vinyl polymer.

6. The method according to claim 2, wherein the tetrabenzodiazadiketoperylene pigment or dye is incorporated into a thermoplastic, elastomeric, crosslinked or inherently crosslinked polymer which is in the form of a film or coating applied to the surface of a substrate, or in the form of a fiber, sheet or other molded or shaped article.

7. An infra red reflective composition having a reflectance of greater than about 50 percent at wavelengths between 800 and 1200 nm, preferably at a wavelength of 1000 nm, which composition comprises a thermoplastic, elastomeric, crosslinked or inherently crosslinked polymer and a tetrabenzodiazadiketoperylene (TBDKP) colourant of formula 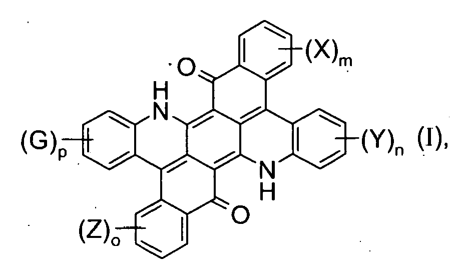 wherein
X, Y, Z and G independently of each other are linear or branched C₁-C₁₂alkyl, C₃-C₆cycloalkyl, C₇-C₁₂aralkyl, C₆-C₁₀aryl, C₃-C₉ saturated or unsaturated heterocycle, halogen, OR, CF₃, COOR, CONR'R", NO₂, NR'R", SO₃H or SO₂NR'R";
R' and R", independently of each other are hydrogen, linear or branched C₁-C₈alkyl, C₃-C₆cydoalkyl; C₆-C₁₀aryl or C₇-C₁₂aralkyl, and when attached to nitrogen, R' and R" can also, together with the nitrogen atom to which they are attached, form a 5-, 6- or 7-membered ring which is uninterrupted or interrupted by O, NH or N(C₁-C₄alkyl);
and m, n, o and p are independently 0, 1, 2, 3 or 4, and when m, n, o or p is 2, 3 or 4, each X, Y, Z or G substituent is independently from all others a group as defined above.

8. An infra red reflective composition according to claim 7, wherein X, Y, Z and G independently of each other are linear or branched C₁-C₁₂alkyl, C₇-C₁₂aralkyl, C₆-C₁₀aryl, F, Cl, Br, I, OR', COOR', CONR'R", NO₂, NR'R", SO₃H or SO₂NR'R" and m, n, o and p are independently 0, 1, 2, 3 or 4, preferably wherein X, Y, Z and G independently of each other are linear or branched C₁-C₁₂alkyl, C₇-C₁₂aralkyl, C₆-C₁₀aryl, F, Cl, Br, OR', COOR', CONR'R", NO₂, NR'R", SO₃H or SO₂NR'R" and m, n, o and p are independently 0, 1 or 2, most preferred wherein the colourant is of formula

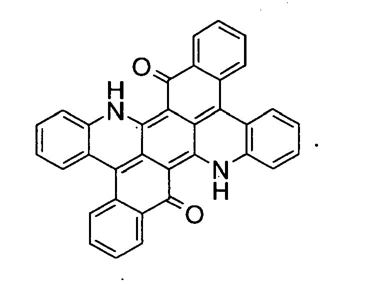

9. An infra red reflective composition of claim 7 or 8, wherein the polymer is a polyolefin, polyamide, polyurethane, polyacrylate, polyacrylamide, polyvinyl alcohol, polycarbonate, polystyrene, polyester, polyacetal, a natural or synthetic rubber or a halogenated vinyl polymer.

10. The composition of claim 7 or 8, which is a coating composition.

11. An article comprising a substrate which is coated with the composition of claim 10.

12. A method for laser welding an article, wherein a TBDKP colourant of formula (I) according to claim 1 is incorporated into a polymeric composition which is in contact with a surface of a meltable substrate containing an IR absorbing material, then IR radiation preferably from a laser of wavelength in the range from 700 to 2000 nm is passed through the layer containing the colourant of formula (1) to the underlying substrate generating enough heat at the point of irradiation to melt together the two materials.

## Patentansprüche

1. Verfahren zur Herstellung eines Infrarot (IR) reflektierenden organischen oder anorganischen Substrats, umfassend die Einarbeitung in das Substrat, oder die Auftragung auf die Oberfläche des Substrats, von einer Zusammensetzung, enthaltend ein Tetrabenzo-diaza-diketoperylen (TBDKP)-Färbemittel der Formel 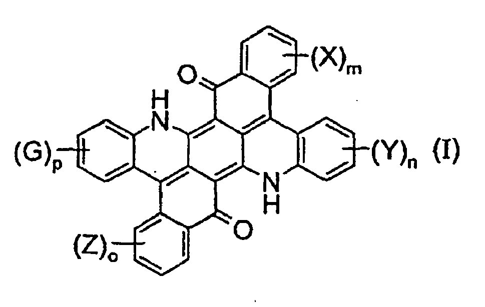
in einer Menge, die wirksam ist, um dem organischen oder anorganischen Substrat ein Infrarot-Reflexionsvermögen von größer als 50 Prozent bei Wellenlängen zwischen 800 und 1200 nm zu verleihen,
wobei in Formel (I)
X, Y, Z und G unabhängig voneinander lineares oder verzweigtes C₁-C₁₂-Alkyl, C₃-C₆-Cycloalkyl, C₇-C₁₂-Aralkyl, C₆-C₁₀-Aryl, gesättigten oder ungesättigten C₃-C₉-Heterocyclus, Halogen, OR, CF₃, COOR, CONR'R", NO₂, NR'R", SO₃H oder SO₂NR'R" darstellen;
R' und R" unabhängig voneinander Wasserstoff, lineares oder verzweigtes C₁-C₈-Alkyl, C₃-C₆-Cycloalkyl, C₆-C₁₀-Aryl oder C₇-C₁₂-Aralkyl darstellen, und wenn an Stickstoff gebunden, R' und R", zusammen mit dem Stickstoff-Atom, an das sie gebunden sind, auch einen 5-, 6- oder 7-gliedrigen Ring, der nicht unterbrochen oder durch O, NH oder N(C₁-C₄-Alkyl) unterbrochen ist, bilden können;
und m, n, o und p unabhängig 0, 1, 2, 3 oder 4 sind, und wenn m, n, o oder p 2, 3 oder 4 ist, jeder Substituent X, Y, Z oder G unabhängig von allen anderen eine wie vorstehend definierte Gruppe darstellt.

2. Verfahren nach Anspruch 1, wobei das Färbemittel der Formel (I) in ein thermoplastisches, elastomeres, vernetztes oder inhärent vernetztes Polymer eingearbeitet wird.

3. Verfahren nach Anspruch 1, wobei X, Y, Z und G unabhängig voneinander lineares oder verzweigtes C₁-C₁₂-Alkyl, C₇-C₁₂-Aralkyl, C₆-C₁₀-Aryl, F, Cl, Br, I, OR', COOR', CONR'R", NO₂, NR'R", SO₃H oder SO₂NR'R" darstellen und m, n, o und p unabhängig 0, 1, 2, 3 oder 4 sind, vorzugsweise wobei X, Y, Z und G unabhängig voneinander lineares oder verzweigtes C₁-C₁₂-Alkyl, C₇-C₁₂-Aralkyl, C₆-C₁₀-Aryl, F, Cl, Br, OR', COOR', CONR'R", NO₂, NR'R", SO₃H oder SO₂NR'R" darstellen und m, n, o und p unabhängig 0, 1 oder 2 sind, besonders bevorzugt, worin das Färbemittel die Formel 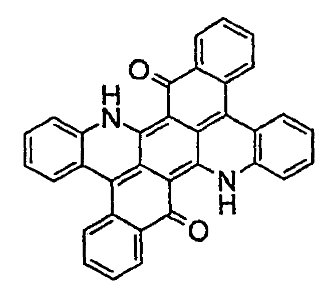 aufweist.

4. Verfahren nach Anspruch 1, wobei das Tetrabenzo-diaza-diketo-perylen in eine Beschichtungszusammensetzung eingearbeitet wird, die auf die Oberfläche von dem Substrat aufgetragen wird.

5. Verfahren nach Anspruch 2, wobei das Tetrabenzo-diaza-diketo-perylen-Färbemittel der Formel (I) ein Pigment darstellt und das Polymer vorzugsweise ein Polyolefin, Polyamid, Polyurethan, Polyacrylat, Polyacrylamid, Polyvinyl-alkohol, Polycarbonat, Polystyrol, Polyester, Polyacetal, einen natürlichen oder synthetischen Kautschuk oder ein halogeniertes Vinyl-Polymer darstellt.

6. Verfahren nach Anspruch 2, wobei das / der Tetrabenzo-diaza-diketo-perylen-Pigment oder -Farbstoff in ein thermoplastisches, elastomeres, vernetztes oder inhärent vernetztes Polymer eingearbeitet wird, das in Form von einem Film oder einer Beschichtung, die auf die Oberfläche von einem Substrat aufgetragen wird, oder in Form von einer Faser, Folie oder anderem geformten oder gestalteten Gegenstand vorliegt.

7. Infrarot-reflektierende Zusammensetzung mit einem Reflexionsvermögen von größer als etwa 50 Prozent bei Wellenlängen zwischen 800 und 1200 nm, vorzugsweise bei einer Wellenlänge von 1000 nm, wobei die Zusammensetzung ein thermoplastisches, elastomeres, vernetztes oder inhärent vernetztes Polymer und ein Tetrabenzo-diaza-diketo-perylen (TBDKP)-Färbemittel der Formel 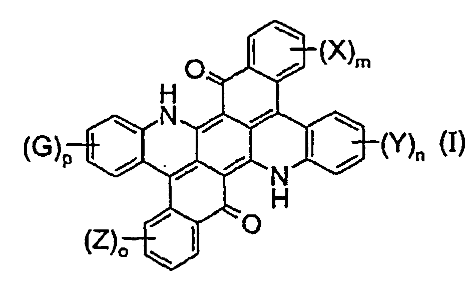 umfasst, worin
X, Y, Z und G unabhängig voneinander lineares oder verzweigtes C₁-C₁₂-Alkyl, C₃-C₆-Cycloalkyl, C₇-C₁₂-Aralkyl, C₆-C₁₀-Aryl, gesättigten oder ungesättigten C₃-C₉-Heterocyclus, Halogen, OR, CF₃, COOR, CONR'R", NO₂, NR'R", SO₃H oder SO₂NR'R" darstellen;
R' und R" unabhängig voneinander Wasserstoff, lineares oder verzweigtes C₁-C₈-Alkyl, C₃-C₆-Cycloalkyl, C₆-C₁₀-Aryl oder C₇-C₁₂-Aralkyl darstellen, und wenn an Stickstoff gebunden, R' und R", zusammen mit dem Stickstoff-Atom, an das sie gebunden sind, auch einen 5-, 6- oder 7-gliedrigen Ring, der nicht unterbrochen oder durch O, NH oder N(C₁-C₄-Alkyl) unterbrochen ist, bilden können;
und m, n, o und p unabhängig 0, 1, 2, 3 oder 4 sind, und wenn m, n, o oder p 2, 3 oder 4 ist, jeder Substituent X, Y, Z oder G unabhängig von allen anderen eine wie vorstehend definierte Gruppe darstellt.

8. Infrarot-reflektierende Zusammensetzung nach Anspruch 7, worin X, Y, Z und G unabhängig voneinander lineares oder verzweigtes C₁-C₁₂-Alkyl, C₇-C₁₂-Aralkyl, C₆-C₁₀-Aryl, F, Cl, Br, I, OR', COOR', CONR'R", NO₂, NR'R", SO₃H oder SO₂NR'R" darstellen und m, n, o und p unabhängig 0, 1, 2, 3 oder 4 sind, vorzugsweise worin X, Y, Z und G unabhängig voneinander lineares oder verzweigtes C₁-C₁₂-Alkyl, C₇-C₁₂-Aralkyl, C₆-C₁₀-Aryl, F, Cl, Br, OR', COOR', CONR'R", NO₂, NR'R", SO₃H oder SO₂NR'R" darstellen und m, n, o und p unabhängig 0, 1 oder 2 sind, besonders bevorzugt, worin das Färbemittel die Formel 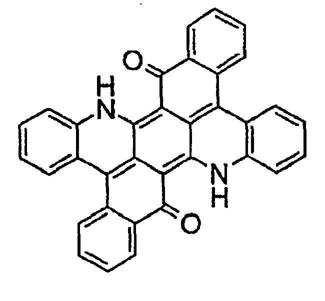 aufweist.

9. Infrarot-reflektierende Zusammensetzung nach Anspruch 7 oder 8, worin das Polymer ein Polyolefin, Polyamid, Polyurethan, Polyacrylat, Polyacrylamid, Polyvinyl-alkohol, Polycarbonat, Polystyrol, Polyester, Polyacetal, ein natürlicher oder synthetischer Kautschuk oder ein halogeniertes Vinyl-Polymer ist.

10. Zusammensetzung nach Anspruch 7 oder 8, die eine Beschichtungszusammensetzung ist.

11. Gegenstand, umfassend ein Substrat, das mit der Zusammensetzung von Anspruch 10 beschichtet ist.

12. Verfahren zum Laser-Schweißen eines Gegenstands, wobei ein TBDKP-Färbemittel der Formel (I) nach Anspruch 1 in eine polymere Zusammensetzung eingearbeitet wird, die in Kontakt mit einer Oberfläche von einem schmelzbaren Substrat ist, welches ein IR absorbierendes Material enthält, dann IR-Strahlung, vorzugsweise von einem Laser einer Wellenlänge in dem Bereich von 700 bis 2000 nm durch die Schicht, die das Färbemittel der Formel (I) enthält, auf das darunter liegende Substrat geleitet wird, wobei ausreichend Wärme an dem Punkt der Bestrahlung erzeugt wird, um die zwei Materialien miteinander zu verschmelzen.

## Revendications

1. Méthode de préparation d'un substrat organique ou inorganique réfléchissant les infra-rouges (IR) comprenant l'incorporation dans le substrat, ou l'application sur la surface du substrat, d'une composition contenant un colorant tétrabenzodiazadicétopérylène (TBDKP) de formule 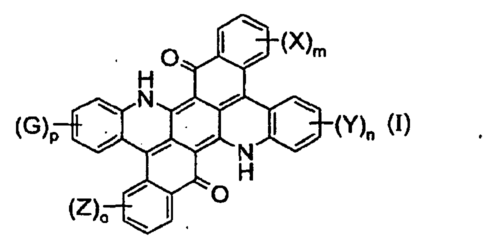
selon une quantité efficace pour procurer au substrat organique ou inorganique un facteur de réflexion infra-rouge supérieur à 50% au niveau des longueurs d'ondes comprise entre 800 et 1 200 nm, **caractérisée en ce que** dans la formule (I)
X, Y, Z et G, indépendamment les uns des autres, sont C₁-C₁₂alkyle linéaire ou ramifié, C₃-C₆cycloalkyle, C₇-C₁₂aralkyle, C₆-C₁₀aryle, C₃-C₉ hétérocycle saturé ou insaturé, halogène, OR, CF₃, COOR, CONR'R", NO₂, NR'R", SO₃H ou SO₂NR'R" ;
R' et R", indépendamment l'un de l'autre, sont hydrogène, C₁-C₈alkyle, C₃-C₆cycloalkyle, C₆-C₁₀aryle ou C₇-C₁₂aralkyle linéaire ou ramifié, et lorsqu'ils sont attachés à azote, R' et R" peuvent également, de pair avec l'atome d'azote auquel ils sont attachés, former un cycle à 5, 6 ou 7 ramifications qui est non interrompu ou interrompu par O, NH ou N(C₁-C₄alkyle) ;
et m, n, o et p sont, indépendamment, 0, 1, 2, 3 ou 4, et lorsque m, n, o ou p est 2, 3 ou 4, chaque substituant X, Y, Z ou G est indépendamment des autres, un groupe tel que défini ci-dessus.

2. Méthode selon la revendication 1, **caractérisée en ce que** le colorant de formule (I) est incorporé dans un polymère thermoplastique, élastomérique, réticulé ou réticulé de façon inhérente.

3. Méthode selon la revendication 1, **caractérisée en ce que** X, Y, Z et G, indépendamment les uns des autres, sont C₁-C₁₂alkyle linéaire ou ramifié, C₇-C₁₂aralkyle, C₆-C₁₀aryle, F, CI, Br, I, OR', COOR', CONR'R", NO₂, NR'R", SO₃H ou SO₂NR'R" et m, n, o et p sont, indépendamment, 0, 1, 2, 3 ou 4, de préférence **caractérisé en ce que** X, Y, Z et G, indépendamment les uns des autres, sont C₁-C₁₂alkyle linéaire ou ramifié, C₇-C₁₂aralkyle, C₆-C₁₀aryle, F, CI, Br, OR', COOR', CONR'R", NO₂, NR'R", SO₃H ou SO₂NR'R" et m, n, o et p sont, indépendamment, 0, 1 ou 2, de préférence **caractérisé en ce que** le colorant est de formule

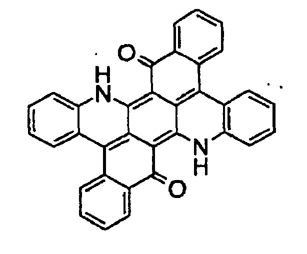

4. Méthode selon la revendication 1, **caractérisée en ce que** le tétrabenzodiazadicétopérylène est incorporé dans une composition de revêtement qui est appliquée sur la surface du substrat.

5. Méthode selon la revendication 2, **caractérisée en ce que** le colorant tétrabenzodiazadicétopérylène de formule (I) est un pigment et **en ce que** le polymère est, de préférence, une polyoléfine, un polyamide, un polyuréthane, un polyacrylate, un polyacrylamide, un alcool polyvinylique, un polycarbonate, un polystyrène, un polyester, un polyacétal, un caoutchouc naturel ou synthétique ou un polymère vinylique halogéné.

6. Méthode selon la revendication 2, **caractérisée en ce que** le pigment ou le colorant tétrabenzodiazadicétopérylène est incorporé dans un polymère thermoplastique, élastomérique, réticulé ou réticulé de façon inhérente qui se présente sous forme d'un film ou d'un revêtement appliqué sur la surface d'un substrat, ou sous forme d'une fibre, d'une feuille ou de tout autre article moulé ou façonné.

7. Composition réfléchissante infra-rouge présentant un facteur de réflexion supérieur à environ 50 % au niveau des longueurs d'ondes comprise entre 800 et 1 200 nm, de préférence, au niveau des longueurs d'ondes de 1 000 nm, ladite composition comprend un polymère thermoplastique, élastomérique, réticulé ou réticulé de façon inhérente et un colorant tétrabenzodiazadicétopérylène (TBDKP) de formule 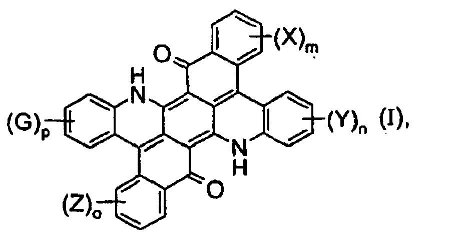 dans laquelle,
X, Y, Z et G, indépendamment les uns des autres, sont C₁-C₁₂alkyle linéaire ou ramifié, C₃-C₆cycloalkyle, C₇-C₁₂aralkyle, C₆-C₁₀aryle, C₃-C₉ hétérocycle saturé ou insaturé, halogène, OR, CF₃, COOR, CONR'R", NO₂, NR'R", SO₃H ou SO₂NR'R" ;
R' et R", indépendamment l'un de l'autre, sont hydrogène, C₁-C₈alkyle, C₃-C₆cycloalkyle, C₆-C₁₀aryle ou C₇-C₁₂aralkyle linéaire ou ramifié, et lorsqu'ils sont attachés à azote, R' et R" peuvent également, de pair avec l'atome d'azote auquel ils sont attachés, former un cycle à 5, 6 ou 7 ramifications qui est non interrompu ou interrompu par O, NH ou N(C₁-C₄alkyle) ;
et m, n, o et p sont, indépendamment, 0, 1, 2, 3 ou 4, et lorsque m, n, o ou p est 2, 3 ou 4, chaque substituant X, Y, Z ou G est indépendamment des autres, un groupe tel que défini ci-dessus.

8. Composition réfléchissante infra-rouge selon la revendication 7, **caractérisée en ce que** X, Y, Z et G, indépendamment les uns des autres, sont C₁-C₁₂alkyle, C₇-C₁₂aralkyle, C₆-C₁₀aryle linéaire ou ramifié, F, CI, Br, I, OR', COOR', CONR'R", NO₂, NR'R", SO₃H ou SO₂NR'R" et m, n, o et p sont, indépendamment, 0, 1, 2, 3 ou 4, de préférence, **caractérisé en ce que** X, Y, Z et G, indépendamment les uns des autres, sont C₁-C₁₂alkyle, C₇-C₁₂aralkyle, C₆-C₁₀aryle linéaire ou ramifié, F, CI, Br, OR', COOR', CONR'R", NO₂, NR'R", SO₃H ou SO₂NR'R" et m, n, o et p sont, indépendamment, 0, 1 ou 2, de préférence, **caractérisé en ce que** le colorant est de formule

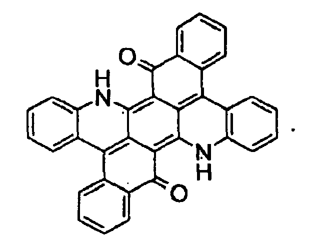

9. Composition réfléchissante infra-rouge selon la revendication 7 ou 8, **caractérisée en ce que** le polymère est une polyoléfine, un polyamide, un polyuréthane, un polyacrylate, un polyacrylamide, un alcool polyvinylique, un polycarbonate, un polystyrène, un polyester, un polyacétal, un caoutchouc naturel ou synthétique ou un polymère vinylique halogéné.

10. Composition selon la revendication 7 ou 8 qui est une composition de revêtement.

11. Article comprenant un substrat qui est revêtu à l'aide de la composition selon la revendication 10.

12. Méthode de soudage au laser d'un article, **caractérisée en ce qu'**un colorant TBDKP de formule (I) selon la revendication 1 est incorporé dans une composition polymérique qui est en contact avec une surface d'un substrat pouvant être fondu contenant un matériau absorbant les IR ; ensuite le rayonnement IR provenant, de préférence d'un laser dont la longueur d'ondes est comprise dans la gamme allant de 700 à 2 000 nm, est passé à travers la couche contenant le colorant de formule (I) vers le substrat sous-jacent générant assez de chaleur au moment du rayonnement pour fondre ensemble les deux matériaux.
